# EUROPÄISCHE PATENTSCHRIFT

(11) **EP 2 729 197 B1**
(45) Veröffentlichungstag und Bekanntmachung des Hinweises auf die Patenterteilung: **26.08.2015**
(21) Anmeldenummer: 12735436.3
(22) Anmeldetag: 05.07.2012
(51) Int. Cl.: A61M 1/00

(54) **THORAXDRAINAGEVORRICHTUNG MIT REDUZIERTEM GEGENDRUCK**
THORACIC DRAINAGE DEVICE HAVING REDUCED COUNTER-PRESSURE
DISPOSITIF DE DRAINAGE THORACIQUE AVEC CONTRE-PRESSION RÉDUITE

(30) Priorität: 07.07.2011 CH 11412011
(43) Veröffentlichungstag der Anmeldung: 14.05.2014
(73) Patentinhaber: Medela Holding AG, 6340 Baar (CH)
(72) Erfinder: EHLERT, Hilmar, CH-6052 Hergiswil (CH)
(74) Vertreter: Detken, Andreas
(86) Internationale Anmeldenummer: PCT/CH2012/000156
(87) Internationale Veröffentlichungsnummer: WO 2013/003970

(56) Entgegenhaltungen:
- WO-A1-2005/061025
- US-A- 4 439 190
- US-A- 4 654 029
- US-A- 5 931 821
- US-A1- 2006 122 558

## Beschreibung

### TECHNISCHES GEBIET

Die vorliegende Erfindung betrifft eine Vorrichtung für die Thoraxdrainage sowie ein entsprechendes Verfahren und Computerprogramm.

### STAND DER TECHNIK

Bei Patienten mit Defekten an der Brustwand oder Lungenoberfläche können sich im Pleuraraum Luft und Sekrete ansammeln. Zur Behandlung wird häufig eine Thoraxdrainage (Pleuraldrainage) durchgeführt, bei der Luft und Sekrete über einen Drainagekatheter aus dem Pleuraraum entfernt werden. Dabei werden aktive Systeme, bei denen aktiv ein Vakuum an den Pleuraraum angelegt wird, und passive Systeme, die ohne Vakuumunterstützung arbeiten, unterschieden.

Bei beiden Arten von Systemen ist der Drainagekatheter meist über eine Drainageleitung mit einem Sekretsammelbehälter verbunden, um flüssige Sekrete abzuscheiden. An den Sekretsammelbehälter schliesst sich meist ein Wasserschloss an, welches die Entlüftung des Sekretsammelbehälters erlaubt, aber verhindert, dass Luft durch den Sekretsammelbehälter, die Drainageleitung und den Drainagekatheter zurück in den Pleuraraum gelangt. Bei aktiven Drainagesystemen ist dann mit dem Wasserschloss zusätzlich eine Vakuumquelle verbunden. Eine solches Thoraxdrainagesystem ist z.B. in der WO 2003/103747 angegeben.

Für aktive Thoraxdrainagesysteme wurde im Stand der Technik schon vorgeschlagen, auf ein Wasserschloss zu verzichten oder das Wasserschloss durch ein mechanisches Einwegventil zu ersetzen, um das Thoraxdrainagesystem leichter handhabbar zu machen und insbesondere tragbar ausgestalten zu können. Beispiele für derartige aktive Thoraxdrainagesysteme finden sich in der WO 99/10024, in der WO 2007/128156 und in der WO 2008/141471.

Ein Wasserschloss besteht aus einem luftdicht verschlossenen und teilweise mit Flüssigkeit, meist Wasser, gefüllten Behälter, in den von oben zwei Röhren hineinragen, die einen ersten und einen zweiten Anschluss des Wasserschlosses bilden. Die erste Röhre ragt dabei bis unter die Wasseroberfläche, während die zweite Röhre oberhalb der Wasseroberfläche endet. Luft kann durch die erste Röhre hindurch ins Wasserschloss eintreten, in Form von Luftblasen durch das Wasser hindurch gelangen und durch die zweite Röhre entweichen. In der umgekehrten Richtung verhindert das Wasser, dass Luft, die durch die zweite Röhre eintritt, in die erste Röhre gelangen kann. Das Wasserschloss wirkt insofern als Einwegventil (Rückschlagventil).

Damit Luft durch die erste Röhre und durch das Wasser hindurch gelangen kann, muss die Luft einen bestimmten Mindestdruck aufweisen. Dieser ist dadurch gegeben, dass die Luft zunächst die Wassersäule im unteren Ende der ersten Röhre verdrängen muss. Typischerweise ragt die erste Röhre zwischen einem und wenigen Zentimetern ins Wasser hinein, z.B. um 2 cm. Die durch die erste Röhre ins Wasserschloss eintretende Luft muss in diesem Fall entsprechend einen positiven Mindestdruck von ca. 2 cm H₂O (entsprechend 2 mbar bzw. 0.2 kPa) gegenüber dem Atmosphärendruck aufweisen, um durch das Wasser hindurch zu gelangen.

Bei einer passiven Thoraxdrainage mit Wasserschloss muss der Patient diesen Mindestdruck selbst aufbringen, um Luft aus dem Pleuraraum auspressen zu können, z.B. durch Husten. Dabei kollabiert die Lunge bis zu einem gewissen Grad. Dies ist dem Heilungsprozess meist nicht förderlich.

Die US 2006/0122558 A1 offenbart eine computergesteuerte, aktive Drainagevorrichtung. Eine Vakuumpumpe saugt Fluide von einem Patienten ab. Die Höhe des Vakuums wird über ein elektronisches Nebenluftventil eingestellt, welches der Vakuumpumpe zusätzliche Luft aus der Atmosphäre zuführt, um die Saugleistung zu regulieren, der der Patient ausgesetzt ist. Ein passiver Betriebsmodus ist nicht vorgesehen.

Die US 4,654,029 offenbart eine elektronisch gesteuerte, aktive Thoraxdrainagevorrichtung. Fluide aus dem Pleuraraum werden durch ein Hospitalvakuumsystem abgesaugt. Zur Regelung der Vakuumhöhe ist in der Absaugleitung ein Saugdrucksensor vorhanden, der mit einem Saugdruckregler zusammenwirkt. Der Saugdruckregler betätigt ein motorisch angetriebenes Nebenluftventil in der Absaugleitung, um die Saugleistung zu regulieren. Um bei einem Ausfall des Hospitalvakuumsystems zu verhindern, dass sich in der Absaugleitung ein übermässiger positiver Druck aufbaut, öffnet der Saugdruckregler die Nebenluftöffnung auch dann, wenn der Druck in der Absaugleitung einen Wert von 1 cm H₂O (entsprechend 1 mbar bzw. 0.1 kPa) überschreitet. Der Saugdrucksensor ist stromabwärts vom Nebenluftventil angeordnet; der vom Saugdrucksensor gemessene Druckwert wird daher durch das Nebenluftventil beeinflusst.

In US 5,931,821 und US 4,439,190 sind Drainagevorrichtungen mit mechanischen Überdruckventilen offenbart.

### DARSTELLUNG DER ERFINDUNG

In einem ersten Aspekt der vorliegenden Erfindung ist es daher eine Aufgabe, eine Vorrichtung zur Thoraxdrainage anzugeben, die im passiven Betrieb das Auspressen von Luft aus dem Pleuraraum durch den Patienten erleichtert.

Diese Aufgabe wird durch eine Vorrichtung mit den Merkmalen des Anspruchs 1 gelöst. Weitere Ausführungsformen sind in den abhängigen Ansprüchen angegeben.

Es wird also eine Vorrichtung für die Thoraxdrainage vorgeschlagen, welche aufweist:
- einen Anschluss für einen mit dem Pleuraraum verbindbaren Sekretsammelbehälter (im Folgenden als Behälteranschluss bezeichnet); und
- eine Entlüftungseinrichtung, um den Sekretsammelbehälter zu entlüften.

Die Entlüftungseinrichtung zeichnet sich erfindungsgemäss dadurch aus, dass sie ein steuerbares erstes Ventil umfasst. Dieses steuerbare Ventil wird im Folgenden auch als Entlüftungsventil bezeichnet.

Die Vorrichtung kann auch den eigentlichen Sekretsammelbehälter mit umfassen; die Erfindung erstreckt sich aber auch auf die Vorrichtung ohne angeschlossenen Sekretsammelbehälter.

Indem die Entlüftungseinrichtung ein steuerbares Ventil aufweist, d.h. ein Ventil, das durch geeignete Steuersignale ohne manuelle Einwirkung seinen Betriebszustand verändern kann, wird es möglich, den Gegendruck beim Entlüften sehr gering zu halten. Dies kann dadurch erreicht werden, dass das Ventil gezielt geöffnet wird, wenn die Differenz zwischen dem Druck im Sekretsammelbehälter und dem Aussendruck einen definierten ersten Schwellwert übersteigt. Dieser Schwellwert kann als ein sehr kleiner positiver Wert gewählt werden, z.B. ein Wert zwischen 0.001 und 0.1 kPa, bevorzugt zwischen 0.01 und 0.05 kPa. Dadurch öffnet das Ventil, sobald Luft vom Pleuraraum in den Sekretsammelbehälter gelangt, ohne dass dazu zunächst ein relativ hoher Gegendruck überwunden werden muss. Dadurch kann der Patient die im Pleuraraum angesammelte Luft leichter auspressen. Sobald der Druck einen zweiten Schwellwert wieder unterschreitet, der gleich oder geringer als der erste Schwellwert sein kann, z.B. wenn die Druckdifferenz negativ wird, kann das Ventil wieder aktiv geschlossen werden, um ein Zurückströmen von Luft in den Pleuraraum zu verhindern.

Das Entlüftungsventil ist bevorzugt durch elektrische Signale steuerbar, kann aber auch durch andere Arten von Signalen, z.B. durch optische Signale, hydraulisch oder pneumatisch steuerbar sein. Dies gilt auch für alle weiteren im Folgenden genannten steuerbaren Ventile. Geeignete steuerbare Ventile sind aus dem Stand der Technik in grosser Zahl bekannt.

Um das Entlüftungsventil automatisch anzusteuern, umfasst die Vorrichtung vorzugsweise einen ersten Drucksensor, um den Druck im Sekretsammelbehälter zu bestimmen, und eine mit diesem Drucksensor zusammenwirkende elektronische Steuereinrichtung (einen Controller), die das Entlüftungsventil unter Berücksichtigung des gemessenen Drucks ansteuert. Der erste Drucksensor wird im Folgenden auch als Behälter-Drucksensor bezeichnet, da er den Druck nahe beim Sekretsammelbehälter misst. Geeignete Drucksensoren sind aus dem Stand der Technik in grosser Zahl bekannt. Die Steuereinrichtung kann mit Bedienelementen, z.B. Bedienknöpfen und/oder einem Display, insbesondere einem berührungsempfindlichen Display (Touchscreen-Display) versehen sein. Sie kann netzbetrieben sein, ist aber bevorzugt mit einer autarken Energiequelle, z.B. in Form von wegwerfbaren oder wiederaufladbaren Batterien, versehen. Dies ermöglicht es, die Vorrichtung tragbar auszugestalten. Die Steuereinrichtung kann einen Speicher, z.B. einen Flashspeicher, umfassen und dazu ausgebildet sein, fortlaufend oder zu bestimmten Zeitpunkten vorbestimmte Betriebsdaten im Speicher abzulegen, insbesondere den gemessenen Druck im Sekretsammelbehälter und/oder den Betriebszustand des Entlüftungsventils. So können z.B. die Zeitpunkte, zu denen das Entlüftungsventil geöffnet und geschlossen wird, abgespeichert werden. Auf diese Weise wird es möglich, den Heilungsverlauf des Patienten zu überwachen (Monitoring).

Zusätzlich kann die Vorrichtung mit einem Durchflusssensor versehen sein, um den aus dem Sekretsammelbehälter durch den Behälteranschluss in die Vorrichtung eintretenden Gasvolumenstrom zu messen. Die Steuereinrichtung ist dann vorzugsweise dazu ausgebildet, den gemessenen Gasvolumenstrom darzustellen und/oder fortlaufend oder zu bestimmten Zeitpunkten im Speicher abzulegen. Auf diese Weise wird es möglich, den Heilungsverlauf noch besser zu überwachen.

Die Vorrichtung kann derart ausgestaltet sein, dass optional ein aktiver Betrieb ermöglicht wird, d.h., dass optional ein Vakuum an den Sekretsammelbehälter anlegbar ist. Dazu kann die Vorrichtung einen Vakuumanschluss zur Verbindung mit einer Vakuumquelle aufweisen. Dabei handelt es sich bevorzugt um eine externe Vakuumquelle, z.B. um ein Spitalvakuumsystem, so dass die eigentliche Vorrichtung sehr kompakt gehalten werden kann. Die Vorrichtung kann aber auch selbst eine interne Vakuumquelle, z.B. eine Saugpumpe, aufweisen. Indem ein Vakuumanschluss vorhanden ist, wird es möglich, Patienten bei Bedarf zunächst mit einer aktiven Thoraxdrainage zu behandeln (z.B. in einer Anfangsphase nach einer Operation) und bei fortgeschrittenem Heilungsverlauf ohne Wechsel der Drainagevorrichtung auf eine passive Thoraxdrainage umzustellen. Gegebenenfalls kann auch eine Umstellung in umgekehrter Richtung, also von passivem Betrieb auf aktiven Betrieb, erfolgen, wenn sich dies als notwendig erweisen sollte. Im aktiven Betrieb kann die Vorrichtung sehr geräuscharm betrieben werden, indem auf ein Wasserschloss verzichtet wird, während bei einem Wasserschloss beim Austritt von Luft jeweils ein (häufig störendes) Blubbern zu hören ist.

Zwischen dem Behälteranschluss und dem Vakuumanschluss ist dazu bevorzugt ein steuerbares zweites Ventil vorgesehen, welches gezielt geöffnet werden kann, um ein am Vakuumanschluss vorliegendes Vakuum an den Sekretsammelbehälter anzulegen. Dieses Ventil wird im Folgenden auch als Vakuumventil bezeichnet. Die Umstellung zwischen passivem und aktivem Betrieb kann dadurch anhand von vorbestimmten Kriterien automatisch durch die Steuereinrichtung erfolgen.

Die Vorrichtung kann ausserdem einen zweiten Drucksensor aufweisen, der dazu ausgebildet ist, den Druck am Vakuumanschluss zu messen. Dieser Drucksensor wird im Folgenden auch als Vakuum-Drucksensor bezeichnet. Die Steuereinrichtung kann dann den vom zweiten Drucksensor gemessenen Druck überwachen. Die Steuereinrichtung kann derart ausgebildet sein, dass sie das zweite Ventil nur dann selbsttätig öffnet, wenn der Druck am Vakuumanschluss gegenüber dem Atmosphärendruck negativ wird bzw. einen bestimmten Grenzwert unterschreitet. Gegebenenfalls öffnet sie dabei das zweite Ventil aber nur dann, wenn weitere Bedingungen erfüllt sind, z.B. wenn der erste Drucksensor oder ein unten noch näher beschriebener Patienten-Drucksensor einen positiven Druck oder einen zu kleinen negativen Druck im Pleuraraum anzeigt, der auf die Notwendigkeit einer aktiven Absaugung hindeutet.

Die Steuereinrichtung kann dazu ausgebildet sein, die Messwerte des zweiten Drucksensors und/oder die Stellung des zweiten Ventils optisch anzuzeigen und/oder fortlaufend oder zu bestimmten Zeitpunkten im Speicher der Steuereinrichtung abzulegen, um eine verbesserte Patientenüberwachung zu gewährleisten.

Das zweite Ventil kann als Stetigventil (Stellventil) ausgebildet sein, welches nicht nur zwei Zustände (geschlossen/geöffnet) einnehmen kann, sondern einen im Wesentlichen kontinuierlich einstellbaren Öffnungsquerschnitt aufweist und dadurch dazu geeignet ist, den am Sekretsammelbehälter anliegenden Vakuumdruck im Wesentlichen kontinuierlich einzustellen. Dadurch wird es möglich, dass die Steuereinrichtung den am Sekretsammelbehälter bzw. am Pleuraraum angelegten Vakuumdruck aktiv regelt. Zur Regelung des Vakuumdrucks kann die Steuereinrichtung insbesondere die vom Behälter-Drucksensor oder von einem unten noch näher beschriebenen Patienten-Drucksensor gemessenen Druckwerte heranziehen. Der optionale Durchflussmesser ist vorzugsweise zwischen dem Behälteranschluss und dem Vakuumventil angeordnet, so dass der durch das Vakuum erzeugte Volumenstrom gemessen werden kann.

Konkret wird die Anordnung der Ventile und Sensoren bevorzugt wie folgt gewählt: Vom Behälteranschluss führt eine Entlüftungsleitung zur Atmosphäre. Das erste Ventil (Entlüftungsventil) ist in dieser Leitung angeordnet, und der erste Drucksensor (Behälter-Drucksensor) misst den Druck in dieser Leitung zwischen dem Behälteranschluss und dem Entlüftungsventil. Der optionale Durchflusssensor ist in dieser Leitung ebenfalls zwischen dem Behälteranschluss und dem Entlüftungsventil angeordnet. Bevorzugt zweigt zudem eine Vakuumleitung, die zum Vakuumanschluss führt, zwischen dem Behälteranschluss und dem Entlüftungsventil von der Entlüftungsleitung ab. Diese Leitung hat bevorzugt einen kleineren Querschnitt als die Entlüftungsleitung, um die maximal mögliche Saugleistung zu begrenzen. Das zweite Ventil (Vakuumventil) und der zweite Drucksensor (Vakuumsensor) sind, soweit vorhanden, in bzw. an dieser Leitung angeordnet. Dadurch kann die Steuerung bzw. Regelung des angelegten Vakuums völlig unabhängig von der oben beschriebenen Steuerung des Entlüftungsventils erfolgen, und das Entlüftungsventil bleibt im aktiven Betriebsmodus permanent geschlossen. Die Vakuumleitung zweigt dabei bevorzugt erst hinter dem Durchflusssensor von der Entlüftungsleitung ab. Dadurch kann auch im aktiven Betriebsmodus der Durchfluss gemessen und gegebenenfalls aufgezeichnet werden.

Optional kann die Vorrichtung zudem einen Füllstandssensor zur Messung des im Sekretsammelbehälter vorhandenen Füllstands (d.h. der angesammelten Sekretmenge) aufweisen. Dabei kann es sich insbesondere um einen kapazitiven Sensor handeln, der an einer zum Sekretsammelbehälter hin weisenden Wand der Vorrichtung untergebracht ist. Dieser Sensor kann ebenfalls mit der Steuereinrichtung verbunden sein. Die Steuereinrichtung ist dann vorzugsweise dazu ausgebildet, ein Signal abzugeben, wenn der Flüssigkeitsstand einen vorbestimmten Wert überschreitet. Die Steuereinrichtung kann alternativ oder zusätzlich dazu ausgebildet sein, den Flüssigkeitsstand optisch anzuzeigen, z.B. auf einer Skala oder auf andere geeignete Weise in einem Display der Steuereinrichtung, und/oder den gemessenen Flüssigkeitsstand fortlaufend oder zu bestimmten Zeitpunkten im Speicher abzulegen.

Thoraxdrainagen werden heutzutage meist mit einem Doppelschlauchsystem durchgeführt. Dabei dient ein erster Schlauch als Drainageleitung, um Luft und Sekrete vom Katheter zum Sekretsammelbehälter zu befördern. Ein zweiter Schlauch dient als Hilfsleitung. Diese Hilfsleitung mündet am patientenseitigen (proximalen) Ende in die Drainageleitung. Die Hilfsleitung dient dann in der Regel dazu, den Druck im Pleuraraum zu messen, ohne dass diese Messung durch Siphoneffekte in der Drainageleitung verfälscht wird. Zudem kann die Hilfsleitung dazu eingesetzt werden, die Drainageleitung zu spülen, wenn es zu Verstopfungen der Drainageleitung kommen sollte.

Zur Verwendung mit einem Doppelschlauchsystem kann die Vorrichtung zusätzlich zum Behälteranschluss auch einen Anschluss für eine solche Hilfsleitung aufweisen. Mit diesem Anschluss ist dann ein dritter Drucksensor verbunden. Dieser Drucksensor wird im Folgenden auch als Patienten-Drucksensor bezeichnet, weil er es ermöglicht, die tatsächlichen Druckverhältnisse im Pleuraraum des Patienten zu messen. Dieser Drucksensor ist dann vorzugsweise wiederum mit der Steuereinrichtung verbunden. Die Steuereinrichtung kann die von diesem Sensor gemessenen Druckwerte wiederum fortlaufend oder zu bestimmten Zeitpunkten speichern und/oder darstellen. Bevorzugt überwacht die Steuereinrichtung die vom Patienten-Drucksensor gemessenen Druckwerte und vergleicht sie fortlaufend oder zu bestimmten Zeitpunkten mit den vom Behälter-Drucksensor gemessenen Druckwerten. Falls diese Werte um mehr als einen vorgegebenen Betrag voneinander abweichen, deutet dies auf eine Verstopfung der Drainageleitung hin, und die Steuereinrichtung kann ein entsprechendes Signal abgeben. Dieses Signal kann insbesondere dazu dienen, einen automatischen Spülvorgang auszulösen. Der Patienten-Drucksensor kann, wie schon erwähnt, auch dazu herangezogen werden, den Vakuumdruck im Pleuraraum zu regeln.

Der Anschluss für die Hilfsleitung ist vorzugsweise ausserdem mit einem dritten steuerbaren Ventil verbunden, das im Folgenden als Spülventil bezeichnet wird. Dieses Ventil schliesst im geschlossenen Zustand den Anschluss für die Hilfsleitung geräteseitig zur Atmosphäre hin ab. Wenn ein Vakuumanschluss vorhanden ist, kann der Spülvorgang dann wie folgt ausgeführt werden. Die Steuereinrichtung öffnet einerseits das Spülventil und andererseits das Vakuumventil. Dadurch entsteht ein Vakuum in der Drainageleitung und somit auch in der Hilfsleitung. Dieses führt dazu, dass Luft von der Atmosphäre durch das Spülventil in die Hilfsleitung eingesaugt wird und durch die Drainageleitung zur Vakuumquelle gelangt. Dadurch wird die Drainageleitung gespült, wie dies grundsätzlich z.B. auch in der WO 2005/061025 angegeben ist.

Bevorzugt sind die genannten Ventile, die genannten Sensoren und die Steuereinrichtung in einem gemeinsamen Gehäuse untergebracht. Der Sekretsammelbehälter ist bevorzugt lösbar mit diesem Gehäuse verbindbar, z.B. über eine Schnappverbindung. Für den Anschluss der Drainageleitung und der optionalen Hilfsleitung kann zudem ein am Gehäuse lösbar anbringbares Anschlussteil vorgesehen sein. Die Verbindung zwischen der Drainageleitung bzw. Hilfsleitung und dem Sekretsammelbehälter bzw. dem Gehäuse kann z.B. entsprechend der WO 2007/128156 oder der WO 2008/141471 ausgestaltet sein. Insgesamt ergibt sich so ein kostengünstig herstellbares, kompaktes System, das tragbar ausgestaltet sein kann. Je nach Bedarf können unterschiedlich grosse Sekretsammelbehälter an das Gehäuse gekoppelt werden. Der Austausch der (bevorzugt wegwerfbaren) Sekretsammelbehälter kann dabei auf einfache und den Patienten nicht beeinträchtigende Weise durchgeführt werden.

Die Steuerungseinrichtung weist vorzugsweise einen digitalen Prozessor und einen Speicher auf, in dem ein Computerprogramm gespeichert ist, welches bei Ausführung durch den Prozessor bewirkt, dass die Steuerungseinrichtung die oben genannten Schritte ausführt.

In einem zweiten Aspekt bezieht sich die vorliegenden Erfindung daher auch auf ein Computerprogramm nach Anspruch 14. Es wird also ein Computerprogramm zur Steuerung einer Thoraxdrainagevorrichtung mit einem Sekretsammelbehälter, mindestens einem steuerbaren ersten Ventil, mindestens einem ersten Drucksensor und einer mit dem ersten Ventil und dem ersten Drucksensor zusammenwirkenden Steuervorrichtung angegeben, wobei das Computerprogramm bei seiner Ausführung die Steuervorrichtung veranlasst, die folgenden Schritte auszuführen:
- Auslesen des ersten Drucksensors, um einen Druck im Sekretsammelbehälter zu ermitteln; und
- Ansteuern des ersten Ventils, um den Sekretsammelbehälter zu entlüften, wenn der ermittelte Druck einen ersten Schwellwert übersteigt.

Wenn ein Vakuumanschluss vorhanden ist, veranlasst das Computerprogramm bei seiner Ausführung die Steuervorrichtung vorzugsweise, ausserdem die folgenden Schritte auszuführen:
- Ermitteln der Differenz zwischen dem Druck an einem Vakuumanschluss der Thoraxdrainagevorrichtung und dem Atmosphärendruck;
- selbsttätiges Öffnen eines zwischen dem Sekretbehälter und dem Vakuumanschluss angeordneten, steuerbaren zweiten Ventils, wenn die genannte Differenz einen vorbestimmten Grenzwert unterschreitet und optional weitere Kriterien erfüllt sind.

Wenn schliesslich auch noch ein Patienten-Drucksensor und ein Spülventil wie oben beschrieben vorhanden sind, kann das Computerprogramm bei seiner Ausführung die Steuervorrichtung veranlassen, den oben genannten Spülvorgang auszuführen.

Weitere Fortbildungen des Computerprogramms sind in den abhängigen Ansprüchen angegeben. Solche Fortbildungen ergeben sich ausserdem sinngemäss aus den obigen

### Ausführungen zur Vorrichtung.

Das Computerprogramm kann insbesondere in Form eines Computerprogrammprodukts auf einem geeigneten Datenträger vorliegen, z.B. auf einer CD-ROM, auf einem Flashspeicher usw., oder über ein Netzwerk zum Download bereitgestellt werden. Es kann in beliebiger Form vorliegen, z.B. als Source Code, Object Code oder Maschinencode.

Beschrieben wird auch ein Verfahren zum Betrieb einer Thoraxdrainagevorrichtung mit einem Sekretsammelbehälter, welches die folgenden Schritte umfasst:
- Ermitteln eines Drucks im Sekretsammelbehälter;
- Entlüften des Sekretsammelbehälters mittels eines steuerbaren Ventils, wenn der Druck einen ersten Schwellwert übersteigt.

Das Verfahren kann ausserdem umfassen:
- Ermitteln der Differenz zwischen dem Druck an einem Vakuumanschluss der Thoraxdrainagevorrichtung und dem Atmosphärendruck;
- selbsttätiges Öffnen eines zwischen dem Sekretbehälter und dem Vakuumanschluss angeordneten, steuerbaren zweiten Ventils, wenn die genannte Differenz einen vorbestimmten Grenzwert unterschreitet und optional weitere Kriterien erfüllt sind. Zudem kann das Verfahren den oben schon beschriebenen Spülvorgang umfassen. Weitere Fortbildungen dieses Verfahrens ergeben sich ebenfalls sinngemäss aus den obigen Ausführungen zur Vorrichtung.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Fig. 1: eine Prinzipskizze eines bevorzugten Ausführungsbeispiels der vorliegenden Erfindung;
- Fig. 2: eine schematische Ansicht einer Steuereinrichtung;
- Fig. 3: eine schematische Ansicht einer erfindungsgemässen Drainagevorrichtung mit einem ersten, kleinen Sekretsammelbehälter;
- Fig. 4: eine Variante der Drainagevorrichtung der Fig. 3;
- Fig. 5: eine schematische Ansicht einer erfindungsgemässen Drainagevorrichtung mit einem zweiten, grösseren Sekretsammelbehälter;
- Fig. 6: ein Diagramm, das einen typischen zeitlichen Verlauf des Pleuraldrucks bei Verwendung einer Thoraxdrainagevorrichtung des Standes der Technik darstellt; und
- Fig. 7: ein Diagramm, das einen typischen zeitlichen Verlauf des Pleuraldrucks bei Verwendung einer erfindungsgemässen Thoraxdrainagevorrichtung darstellt.

### BESCHREIBUNG BEVORZUGTER AUSFÜHRUNGSFORMEN

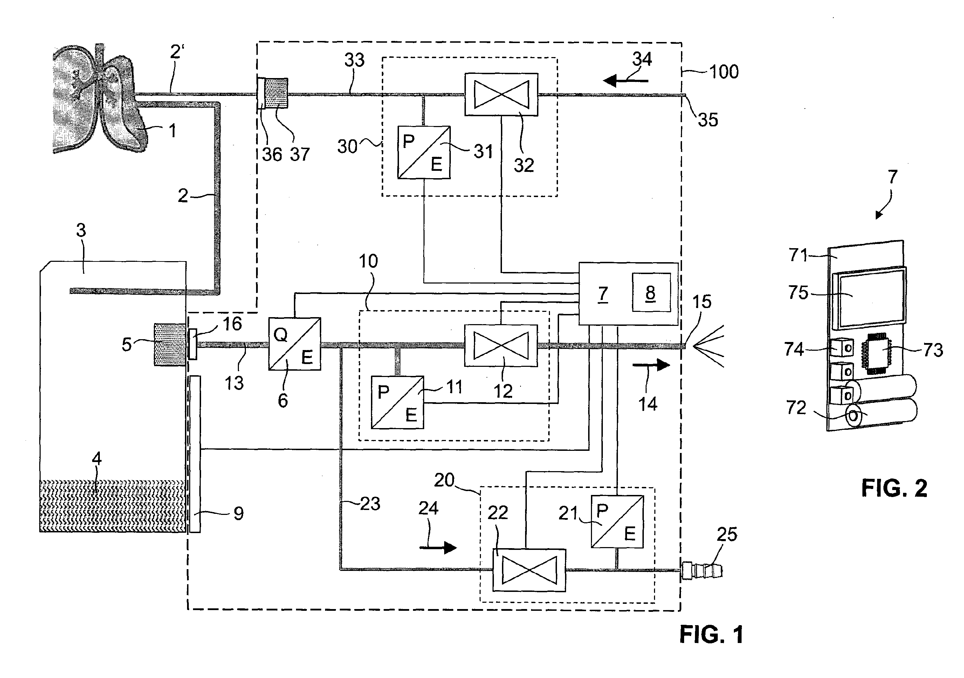

In der Fig. 1 ist eine Prinzipskizze einer erfindungsgemässen Thoraxdrainagevorrichtung 100 dargestellt. Der Pleuraraum 1 eines Patienten ist über einen nicht dargestellten Drainagekatheter und einer daran angeschlossenen Drainageleitung 2 mit einem Sekretsammelbehälter 3 verbunden. Dieser dient dazu, flüssige und gasförmige Bestandteile zu trennen. Die flüssigen Sekrete 4 sammeln sich am Boden des Sekretsammelbehälters 3, während die gasförmigen Bestandteile (im Wesentlichen Luft) durch einen Filter 5 hindurch über einen Behälteranschluss 16 in eine Entlüftungsleitung 13 gelangen. In der Entlüftungsleitung 13 sind bezüglich der Strömungsrichtung 14 hintereinander zunächst ein Durchflusssensor 6 und anschliessend ein elektrisch steuerbares Entlüftungsventil 12 angeordnet. Das Entlüftungsventil 12 gibt im geöffneten Zustand die Entlüftungsleitung 13 an einem Auslass 15 zur Atmosphäre hin frei. Ein Behälter-Drucksensor 11 misst den Druck zwischen dem Durchflusssensor 6 und dem Entlüftungsventil 12. Dieser Drucksensor kann auch vor dem Durchflusssensor 6, z.B. direkt am Behälteranschluss 16 angeordnet sein. Der Behälter-Drucksensor 11 und das Entlüftungsventil 12 bilden gemeinsam eine Entlüftungseinheit 10. Eine Steuereinrichtung 7 mit Speicher 8 liest die Signale der Sensoren 6 und 11 aus und steuert das Ventil 12 an.

Im normalen passiven Betrieb gelangen Luft und Sekrete vom Pleuraraum 1 über die Drainageleitung 2 in den Sekretsammelbehälter 3. Damit die Luft aus dem Sekretsammelbehälter 3 ohne spürbaren Gegendruck entweichen kann, misst die Steuereinrichtung 7 mittels des Behälter-Drucksensors 11 den Druck nahe am Behälteranschluss 16 und öffnet das Entlüftungsventil 12, sobald dieser Druck knapp über dem Atmosphärendruck liegt. Die Ansprechschwelle, bei der das Entlüftungsventil 12 öffnet, kann dabei sehr gering gewählt werden. Sobald der Druck wieder unter den Atmosphärendruck fällt, schliesst die Steuereinrichtung 7 das Entlüftungsventil 12 wieder. Dadurch wird verhindert, dass Luft zurück in den Pleuraraum 1 fliesst. Die Entlüftungseinheit 10 bildet also zusammen mit der Steuereinheit 7 gewissermassen ein elektronisches Rückschlagventil mit sehr geringer Ansprechschwelle.

Im vorliegenden Beispiel ist ein optionaler Füllstandssensor 9 vorhanden, welcher z.B. auf kapazitivem Wege den Füllstand des Sekretsammelbehälters 3 ermittelt und an die Steuereinheit 7 weitergibt. Dadurch können der Patient oder das Pflegepersonal rechtzeitig durch ein z.B. optisches oder akustisches Alarmsignal gewarnt werden, wenn der Sekretsammelbehälter 3 nahezu voll ist.

Optional ist eine Vakuumleitung 23 vorhanden, die von der Entlüftungsleitung 13 zu einem Vakuumanschluss 25 führt. In der Vakuumleitung ist ein elektrisch steuerbares Vakuumventil 22 angeordnet. Ein Vakuumsensor 21 misst den Druck zwischen dem Vakuumanschluss 25 und dem Vakuumventil 22. Der Vakuumsensor 21 und das Vakuumventil 22 bilden dabei eine Vakuum-Steuereinheit 20. Um eine zu starke Saugwirkung zu vermeiden, ist der Querschnitt der Vakuumleitung 23 aus Sicherheitsgründen geringer gewählt als derjenige der Entlüftungsleitung 13.

Auch der Vakuumsensor 21 und das Vakuumventil 22 sind mit der Steuereinrichtung 7 verbunden. Diese stellt fest, ob vom Patienten überhaupt ein Vakuum benötigt wird, d.h. ob die Drainagevorrichtung 100 überhaupt im aktiven Modus betrieben werden soll. Dies kann z.B. durch eine manuelle Eingabe des Pflegepersonals festgelegt werden, z.B. wenn das Pflegepersonal eine Luftfistel feststellt, die durch einen aktiven Betrieb behandelt werden sollte. Ein Wechsel zwischen dem passiven und dem aktiven Betriebsmodus kann aber auch durch die Steuereinrichtung 7 selbsttätig durchgeführt werden, wenn z.B. der vom Behälter-Drucksensor 11 oder vom nachfolgend noch beschriebenen Patienten-Drucksensor 31 gemessene Druck über längere Zeit hinweg sehr nahe beim Atmosphärendruck liegt.

Wenn ein aktiver Betrieb gewünscht ist, ermittelt die Steuereinrichtung 7 mit dem Vakuumsensor 21, ob überhaupt am Vakuumanschluss 25 ein genügendes Vakuum vorhanden ist. Der Vakuumsensor 21 braucht dabei nicht besonders präzise zu sein. Falls dies nicht der Fall ist, gibt die Steuereinrichtung 7 ein entsprechendes Alarmsignal ab, z.B. ein visuelles oder akustisches Signal. Andernfalls öffnet die Steuereinrichtung 7 das Vakuumventil 22.

Die Steuereinrichtung 7 kann dabei derart ausgebildet sein, dass sie den Druck im Sekretsammelbehälter 3 oder im Pleuraraum 1 mit Hilfe des Behälter-Drucksensors 11 und/oder des unten beschriebenen Patienten-Drucksensors 31 selbsttätig auf einen zuvor festgelegten Wert regelt. Da die Vakuumleitung 23 bezüglich der Strömungsrichtung 24 erst hinter dem Durchflusssensor 6 von der Entlüftungsleitung 13 abzweigt, kann die Steuereinrichtung 7 auch im aktiven Betriebsmodus den Durchfluss fortlaufend messen. Die gemessenen Durchflusswerte können dazu herangezogen werden, automatisch wieder auf passiven Betrieb umzustellen, z.B. wenn die Durchflusswerte über einen längeren Zeitraum sehr niedrig bleiben.

Ebenfalls optional ist eine Überwachungs- und Spüleinrichtung 30 vorhanden, die über eine Spülleitung 33, einen Bakterienfilter 37, einen Hilfsanschluss 36 und eine Hilfsleitung 2' mit dem Drainagekatheter und dadurch mit dem Pleuraraum 1 verbunden ist. Die Überwachungs- und Spüleinrichtung 30 umfasst ein Spülventil 32, das im geschlossenen Zustand die Spülleitung 33 zur Atmosphäre hin abschliesst. Ein Patienten-Drucksensor 31 misst den Druck in der Spülleitung 33 zwischen dem Hilfsanschluss 36 und dem Spülventil 32. Der Patienten-Drucksensor 31 und das Spülventil 32 sind wiederum mit der Steuereinrichtung 7 verbunden. Die Steuereinrichtung 7 misst mit dem Patienten-Drucksensor 31 den Druck im Pleuraraum 1. Im aktiven Betriebsmodus nutzt die Steuereinrichtung 7 diese Druckwerte gegebenenfalls zur Regelung des Vakuumdrucks. Zudem vergleicht die Steuereinrichtung 7 periodisch die vom Patienten-Drucksensor 31 ermittelten Druckwerte mit den vom Behälter-Drucksensor 11 ermittelten Druckwerten.

Wenn diese stark voneinander abweichen, weist dies auf eine Funktionsstörung hin, und die Steuereinrichtung gibt ein entsprechendes Alarmsignal ab. Im aktiven Betriebsmodus kann die Steuereinrichtung zudem einen Spülvorgang auslösen. Dazu wird zum einen das Spülventil 32 geöffnet. Zum anderen wird das Vakuumventil 22 weit geöffnet. Dadurch wird Luft über die Hilfsleitung 2' in die Drainageleitung 2 gesaugt und beseitigt dort eventuell vorhandene Verstopfungen.

Sämtliche von der Steuereinrichtung 7 ermittelten Messwerte und sämtliche Betriebszustände können von der Steuereinrichtung fortlaufend oder zu bestimmten Zeitpunkten im Speicher 8 abgelegt werden. Dadurch wird eine lückenlose Überwachung des Heilungsverlaufs möglich. Insbesondere kann der gesamte Verlauf des Luftvolumenstroms sowohl im aktiven als auch im passiven Betriebsmodus aufgezeichnet werden, ebenso der Verlauf der aufgenommenen Sekretmenge und sämtliche Alarmzustände.

Da die Vorrichtung selbst kein Pumpaggregat benötigt, kann sie sehr kompakt ausgebildet werden und benötigt nur wenig Energie. Sie kann daher problemlos auch über längere Zeit durch Batterien versorgt werden. Wegen der geringen Zahl und niedrigen Komplexität der benötigten Bauteile lässt sich die Vorrichtung zudem sehr kostengünstig fertigen. Da kein Wasserschloss und kein Pumpaggregat vorhanden sind, ist die Vorrichtung sehr laufruhig.

In der Fig. 2 ist eine mögliche konkrete Ausgestaltung einer digitalen Steuereinheit 7 illustriert. Die Steuereinheit 7 umfasst eine Platine 71, auf der Batterien 72, ein Prozessor 73 mit integriertem Speicher, Bedienelemente 74 in Form von Tasten und eine visuelle Anzeigeeinrichtung in Form eines Displays 75 montiert sind. Zusätzlich sind nicht dargestellte Analog-Digital-Wandler (ADCs) vorhanden, um die analogen Ausgangssignale der verschiedenen Sensoren in digitale Eingangssignale für den Prozessor 73 umzuwandeln, sowie Treiberschaltkreise, um die Ventile anzusteuern. Im Speicher ist ein Programm abgelegt, das bei seiner Ausführung im Prozessor die Steuereinrichtung 7 veranlasst, die oben genannten Verfahren durchzuführen.

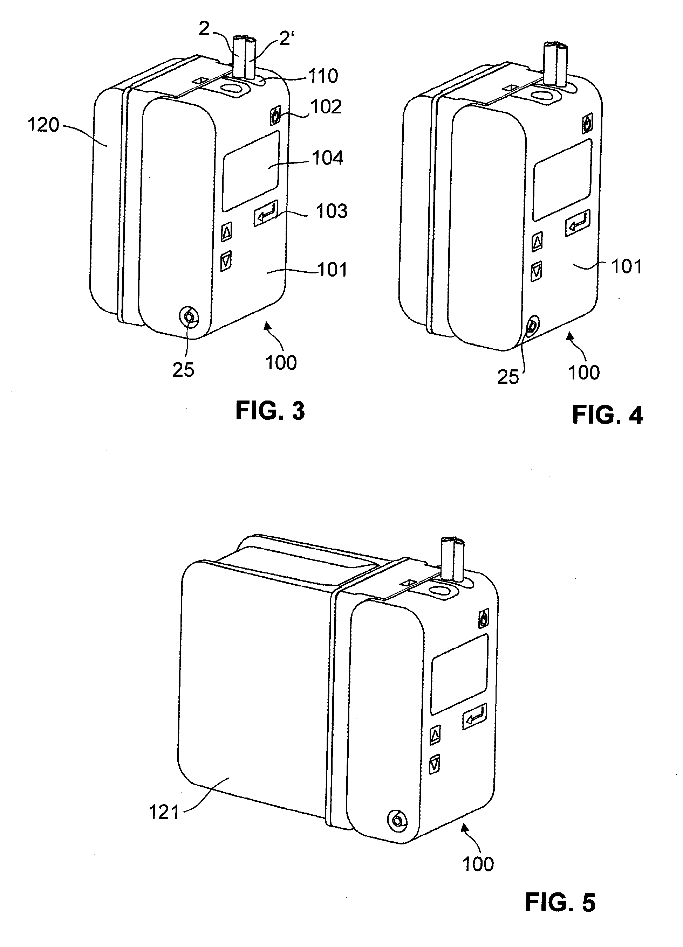

Die Figuren 3-5 illustrieren mögliche Gehäuseformen einer erfindungsgemässen Vorrichtung. Die Vorrichtung 100 der Figuren 3-5 umfasst ein Gehäuse 101, an dessen Vorderseite ein Ein-/Ausschalter 102, Bedienelemente 103 und ein Display 104 vorhanden sind. An der Oberseite ist ein Anschlussteil 110 eingesteckt, mit dem das jeweilige geräteseitige (distale) Ende einer Drainageleitung 2 und einer Hilfsleitung 2' verbunden sind. An der Rückseite des Gehäuses 101 ist ein Sekretsammelbehälter 120 angeklickt, der im Beispiel der Figuren 3 und 4 ein Auffangvolumen von ca. 0.3 1 aufweist. Stattdessen kann aber auch, wie in Fig. 5 gezeigt, ein grösserer Sekretsammelbehälter 121, z.B. mit einem Auffangvolumen von 0.8 1, angeschlossen werden. Das Anschlussteil 110 verbindet die Drainageleitung 2 mit dem Sekretsammelbehälter 120, während es die Hilfsleitung 2' direkt mit den entsprechenden Komponenten in der Vorrichtung 100 verbindet. In einer Seitenwand (Fig. 3 und 5) oder in der vorderen Stirnwand (Fig. 4) des Gehäuses ist ein Vakuumanschluss 25 zum Anschluss an ein Hausvakuum eines Spitals oder an eine externe Vakuumpumpe ausgebildet.

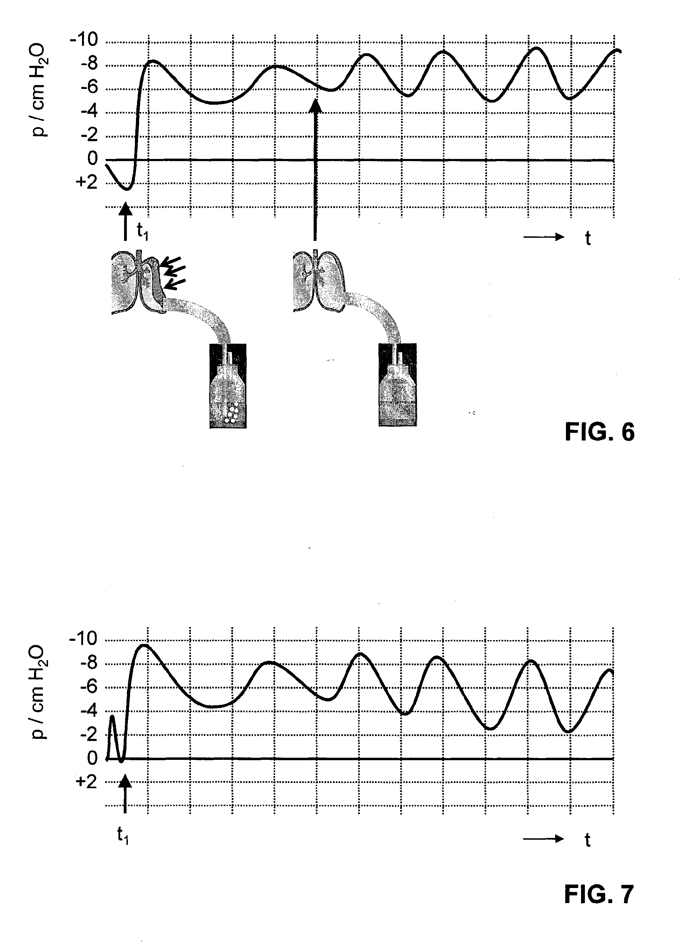

Die Figuren 6 und 7 illustrieren im Vergleich den Druckverlauf im Pleuraraum bei einer traditionellen passiven Thoraxdrainagevorrichtung mit Wasserschloss (Fig. 6) und bei einer Thoraxdrainagevorrichtung gemäss der vorliegenden Erfindung (Fig. 7).

Bei der traditionellen Thoraxdrainagevorrichtung (Fig. 6) muss der Patient zum Zeitpunkt t₁ zunächst die Wassersäule in der Eingangsröhre des Wasserschlosses überwinden, um Luft aus dem Pleuraraum herauszudrücken, z.B. durch Husten. Dazu ist ein positiver Gegendruck von hier ca. 2 cm H₂O (0.2 kPa) nötig (Illustration unten links in Fig. 6). Dies führt zu einem teilweisen Kollaps der Lunge und ist der Heilung nicht förderlich. Zu einem späteren Zeitpunkt t₂ weist dann der Druck im Pleuraraum relativ zum Atmosphärendruck wieder negative Werte auf, die je nach Phase der Atmung (Inspiration/Exspiration) typischerweise zwischen -0.5 kPa und -0.8 kPa liegen. Dies äussert sich in einer positiven Wassersäule in der Eingangsröhre des Wasserschlosses (Illustration unten Mitte in Fig. 6).

Bei der Thoraxdrainagevorrichtung gemäss der vorliegenden Erfindung spricht dagegen das elektronische Einwegventil schon bei sehr geringem positivem Druck gegenüber dem Atmosphärendruck an. Dadurch wird nie ein signifikanter Gegendruck aufgebaut, Der Patient kann dadurch mühelos Luft aus dem Pleuraraum auspressen, ohne dabei die Lunge mehr als nötig zu kollabieren.

### BEZUGSZEICHENLISTE

- 1: Pleuraraum
- 2: Drainageleitung
- 2': Hilfsschlauch
- 3: Sekretsammelbehälter
- 4: Sekret
- 5: Filter
- 6: Durchflusssensor
- 7: Steuereinrichtung
- 8: Speicher
- 9: Füllstandssensor
- 10: Entlüftungseinrichtung
- 11: erster Drucksensor (Behälter-Drucksensor)
- 12: erstes Ventil (Entlüftungsventil)
- 13: Entlüftungsleitung
- 14: Strömungsrichtung
- 15: Auslass
- 16: Behälteranschluss
- 20: Vakuum-Steuereinrichtung
- 21: zweiter Drucksensor (Vakuum-Drucksensor)
- 22: zweites Ventil (Vakuumventil)
- 23: Vakuumleitung
- 24: Strömungsrichtung
- 25: Vakuumanschluss
- 30: Überwachungs-/Spüleinrichtung
- 31: dritter Drucksensor (Patienten-Drucksensor)
- 32: drittes Ventil (Spülventil)
- 33: Mess- und Spülleitung
- 34: Strömungsrichtung
- 35: Einlass
- 36: Hilfsanschluss
- 37: Bakterienfilter
- 71: Platine
- 72: Batterie
- 73: Prozessor/Speicher
- 74: Bedienelement
- 75: Display
- 100: Drainagevorrichtung
- 101: Gehäuse
- 102: Ein-/Ausschalter
- 103: Bedienelement
- 104: Display (Anzeigeeinheit)
- 110: Anschlussteil
- 120: Sekretsammelbehälter (klein)
- 121: Sekretsammelbehälter (gross)
- p: Druck
- t: Zeit
- t₁, t₂: Zeitpunkte

## Patentansprüche

1. Vorrichtung für die Thoraxdrainage, welche aufweist:
- einen Behälteranschluss (16) für einen mit dem Pleuraraum (1) eines Patienten verbindbaren Sekretsammelbehälter (3);
- eine vom Behälteranschluss (16) zur Atmosphäre führende Entlüftungsleitung (13),
- eine Entlüftungseinrichtung (10) mit einem steuerbaren ersten Ventil (12), das in der Entlüftungsleitung angeordnet ist, um den Sekretsammelbehälter (3) zu entlüften, und mit einem ersten Drucksensor (11), um einen Druck im Sekretsammelbehälter (3) zu messen, wobei der erste Drucksensor (11) den Druck in der Entlüftungsleitung (13) zwischen dem Behälteranschluss (16) und dem ersten Ventil (12) misst.; sowie
- eine mit dem ersten Drucksensor (11) zusammenwirkende Steuereinrichtung (7) **dadurch gekennzeichnet, dass** die Steuereinrichtung (7) dazu ausgebildet ist, das erste Ventil (12) zu öffnen, wenn die Differenz zwischen dem Druck im Sekretsammelbehälter (3) und dem Atmosphärendruck einen positiven ersten Schwellwert übersteigt.

2. Vorrichtung nach Anspruch 1, wobei der erste Schwellwert zwischen 0.001 und 0.05 kPa liegt.

3. Vorrichtung nach Anspruch 1 oder 2, wobei die Steuereinrichtung (7) einen Speicher (8) aufweist und dazu ausgebildet ist, vorbestimmte Betriebsdaten im Speicher (8) abzulegen, wobei die Betriebsdaten den gemessenen Druck im Sekretsammelbehälter (3) und/oder einen Betriebszustand des ersten Ventils (12) umfassen.

4. Vorrichtung nach einem der vorhergehenden Ansprüche, welche ausserdem einen Durchflusssensor (6) aufweist, der zwischen dem Behälteranschluss (16) und dem ersten Ventil (12) angeordnet ist und dazu ausgebildet ist, den durch den Behälteranschluss (16) eintretenden Gasvolumenstrom zu messen.

5. Vorrichtung nach einem der vorhergehenden Ansprüche, welche ausserdem einen Vakuumanschluss (25) zur Verbindung mit einer Vakuumquelle aufweist.

6. Vorrichtung nach Anspruch 5, welche zwischen dem Behälteranschluss (16) und dem Vakuumanschluss (25) ein steuerbares zweites Ventil (22) aufweist, um gezielt ein Vakuum an den Sekretsammelbehälter (3) anzulegen.

7. Vorrichtung nach Anspruch 5 oder 6, welche ausserdem einen zweiten Drucksensor (21) aufweist, der dazu ausgebildet ist, den Druck am Vakuumanschluss (25) zu messen.

8. Vorrichtung nach Anspruch 7, wobei die Steuereinrichtung (7) derart ausgebildet ist, dass sie das zweite Ventil (22) selbsttätig öffnet, wenn die Differenz zwischen dem Druck am Vakuumanschluss (25) und dem Atmosphärendruck einen vorbestimmten Grenzwert unterschreitet.

9. Vorrichtung nach einem der Ansprüche 5-8, aufweisend eine Vakuumleitung (23), die zu dem Vakuumanschluss (25) führt und zwischen dem Behälteranschluss (16) und dem ersten Ventil (12) von der Entlüftungsleitung (13) abzweigt.

10. Vorrichtung nach Anspruch 9, wobei die Vakuumleitung (23) einen kleineren Querschnitt als die Entlüftungsleitung (13) aufweist.

11. Vorrichtung nach einem der vorhergehenden Ansprüche, welche ausserdem einen Anschluss für eine Hilfsleitung (33) und einen damit verbundenen dritten Drucksensor (31) aufweist.

12. Vorrichtung nach Anspruch 11, welche ausserdem ein mit dem Anschluss für die Hilfsleitung (33) verbundenes steuerbares drittes Ventil (32) aufweist, das im geschlossenen Zustand den Anschluss für die Hilfsleitung (33) geräteseitig zur Atmosphäre hin abschliesst.

13. Vorrichtung nach einem der vorhergehenden Ansprüche, umfassend ein Gehäuse (101), in dem die Entlüftungseinrichtung (10) untergebracht ist, und mit dem lösbar ein Sekretsammelbehälter (3) verbindbar ist.

14. Computerprogramm zur Steuerung einer Thoraxdrainagevorrichtung mit einem Sekretsammelbehälter (3), mindestens einem steuerbaren ersten Ventil (12), mindestens einem ersten Drucksensor (11) und einer Steuervorrichtung (7), wobei das Computerprogramm bei seiner Ausführung die Steuervorrichtung (7) veranlasst, die folgenden Schritte auszuführen:
- Auslesen des ersten Drucksensors (11), um einen Druck im Sekretsammelbehälter (3) zu ermitteln; und
**gekennzeichnet durch** den Schritt:
- Ansteuern des ersten Ventils (12), um den Sekretsammelbehälter (3) zu entlüften, wenn die Differenz zwischen dem Druck im Sekretsammelbehälter (3) und dem Atmosphärendruck einen positiven ersten Schwellwert übersteigt, wobei der Schwellwert zwischen 0.001 und 0.05 kPa liegt.

15. Computerprogramm nach Anspruch 14, wobei das Computerprogramm bei seiner Ausführung die Steuervorrichtung (7) veranlasst, ausserdem die folgenden Schritte auszuführen:
- Ermitteln der Differenz zwischen dem Druck an einem Vakuumanschluss (25) der Thoraxdrainagevorrichtung und dem Atmosphärendruck;
- selbsttätiges Öffnen eines zwischen dem Sekretbehälter und dem Vakuumanschluss angeordneten, steuerbaren zweiten Ventils (22), wenn die genannte Differenz einen vorbestimmten Grenzwert unterschreitet.

## Claims

1. A device for thoracic drainage, comprising:
- a container connector (16) for a secretion collection container (3) that is adapted to be connected to the pleural cavity (1) of a patient;
- a vent line (13) leading from the container connector (16) to the atmosphere,
- a venting device (10) comprising a controllable first valve (12) arranged in the vent line, for releasing air from the secretion collection container (3), and comprising a first pressure sensor (11) for measuring a pressure in the secretion collection container (3), wherein the first pressure sensor (11) measures a pressure in the vent line (13) between the container connector (16) and the first valve (12); and
- a control device (7), which cooperates with the first pressure sensor (11),
**characterised in that** the control device (7) is configured to open the first valve (12) when a difference between the pressure in the secretion collection container (3) and atmospheric pressure exceeds a positive first threshold value.

2. The device according to Claim 1, wherein the first threshold value is between 0.001 and 0.05 kPa.

3. The device according to Claim 1 or 2, wherein the control device (7) comprises a memory (8) and is configured to store predetermined operating data in the memory (8), wherein the operating data include the pressure measured in the secretion collection container (3) and/or an operating state of the first valve (12).

4. The device according to any one of the preceding claims, further comprising a flow sensor (6), the flow sensor (6) being arranged between the container connector (16) and the first valve (12) and being configured to measure a volumetric flow of gas entering through the container connector (16).

5. The device according to any one of the preceding claims, further comprising a vacuum connector (25) for connection to a vacuum source.

6. The device according to Claim 5, comprising a controllable second valve (22) between the container connector (16) and the vacuum connector (25) in order to selectively apply a vacuum to the secretion collection container (3).

7. The device according to Claim 5 or 6, further comprising a second pressure sensor (21), which is configured to measure a pressure at the vacuum connector (25).

8. The device according to Claim 7, wherein the control device (7) is configured to automatically open the second valve (22) when a difference between a pressure at the vacuum connector (25) and atmospheric pressure falls below a predetermined limit value.

9. The device according to any one of Claims 5-8, comprising a vacuum line (23), which leads to the vacuum connector (25) and branches off from the vent line (13) between the container connector (16) and the first valve (12).

10. The device according to Claim 9, wherein the vacuum line (23) has a smaller cross section than the vent line (13).

11. The device according to any one of the preceding claims, further comprising a connector for an ancillary line (33) and, connected to the connector, a third pressure sensor (31).

12. The device according to Claim 11, further comprising a controllable third valve (32), which is connected to the connector for the ancillary line (33) and by means of which, in the closed state, the connector for the ancillary line (33) is closed off with respect to the atmosphere.

13. The device according to any one of the preceding claims, comprising a housing (101) in which the venting device (10) is accommodated and which is configured to releasably connect to a secretion collection container (3).

14. A computer program for controlling a thoracic drainage device comprising a secretion collection container (3), at least one controllable first valve (12), at least one first pressure sensor (11), and a control device (7), wherein the computer program, when executed, causes the control device (7) to carry out the following steps:
- reading out the first pressure sensor (11) in order to determine a pressure in the secretion collection container (3); and
**characterised by** the step of:
- controlling the first valve (12) in order to release air from the secretion collection container (3) when the difference between the pressure in the secreation collection container (3) and atmospheric pressure exceeds a positive first threshold value, the threshold value being between 0.001 and 0.05 kPa.

15. The computer program according to Claim 14, wherein the computer program, when executed, causes the control device (7) to additionally carry out the following steps:
- determining the difference between a pressure at a vacuum connector (25) of the thoracic drainage device and atmospheric pressure;
- automatically opening a controllable second valve (22), arranged between the secretion container and the vacuum connector, when said difference falls below a predetermined limit value.

## Revendications

1. Dispositif de drainage thoracique comprenant :
- un connecteur de récipient (16) destiné à un récipient de collecte de sécrétion (3) pouvant être relié à la cavité pleurale (1) d'un patient ;
- une conduite de ventilation (13) allant du connecteur de récipient (16) à l'atmosphère,
- un dispositif de ventilation (10) comportant une première soupape (12) pouvant être commandée qui est disposée dans la conduite de ventilation pour éliminer l'air du récipient de collecte de sécrétion (3) et comportant un premier capteur de pression (11) destiné à mesurer une pression régnant dans le récipient de collecte de sécrétion (3), dans lequel le premier capteur de pression (11) mesure la pression régnant dans la conduite de ventilation (13) entre le connecteur de récipient (16) et la première soupape (12) ; et
- un dispositif de commande (7) coopérant avec le premier capteur de pression (11), **caractérisé en ce que** le dispositif de commande (7) est conçu pour ouvrir la première soupape (12) lorsque la différence entre la pression régnant dans le récipient de collecte de sécrétion (3) et la pression atmosphérique dépasse une première valeur de seuil positive.

2. Dispositif selon la revendication 1, dans lequel la première valeur de seuil se situe entre 0,001 et 0,05 kPa.

3. Dispositif selon la revendication 1 ou 2, dans lequel le dispositif de commande (7) comporte une mémoire (8) et est conçu pour stocker des données de fonctionnement prédéterminées dans la mémoire (8), dans lequel les données de fonctionnement comprennent la pression mesurée dans le récipient de collecte de sécrétion (3) et/ou un état de fonctionnement de la première soupape (12).

4. Dispositif selon l'une quelconque des revendications précédentes, comportant en outre un capteur de débit (6) qui est disposé entre le connecteur de récipient (16) et la première soupape (12) et est conçu pour mesurer le débit volumique de gaz pénétrant par l'intermédiaire du connecteur de récipient (16).

5. Dispositif selon l'une quelconque des revendications précédentes, comportant en outre un connecteur à vide (25) destiné à être relié à une source de vide.

6. Dispositif selon la revendication 5, comportant une deuxième soupape (22) pouvant être commandée entre le connecteur de récipient (16) et le connecteur à vide (25) afin d'appliquer un vide de manière ciblée au récipient de collecte de sécrétion (3).

7. Dispositif selon la revendication 5 ou 6, comportant en outre un deuxième capteur de pression (21) qui est conçu pour mesurer la pression au niveau du connecteur à vide (25).

8. Dispositif selon la revendication 7, dans lequel le dispositif de commande (7) est conçu de manière à ce que la deuxième soupape (22) s'ouvre automatiquement lorsque la différence entre la pression au niveau du connecteur à vide (25) et la pression atmosphérique s'abaisse en dessous d'une valeur limite prédéterminée.

9. Dispositif selon l'une quelconque des revendications 5-8, comportant une conduite à vide (23) qui est reliée au connecteur à vide (25) et qui bifurque à partir de la conduite de ventilation (13) entre le connecteur de récipient (16) et la première soupape (12).

10. Dispositif selon la revendication 9, dans lequel la conduite à vide (23) présente une section transversale inférieure à celle de la conduite de ventilation (13).

11. Dispositif selon l'une quelconque des revendications précédentes, comportant en outre un connecteur destiné à une conduite auxiliaire (33) et un troisième capteur de pression (31) relié à celle-ci.

12. Dispositif selon la revendication 11, comportant en outre une troisième soupape (32) pouvant être commandée et reliée au connecteur destiné à la conduite auxiliaire (33), laquelle troisième soupape, dans son état fermé, ferme le raccordement à l'atmosphère destiné à la conduite auxiliaire (33) du côté de l'appareil.

13. Dispositif selon l'une quelconque des revendications précédentes, comprenant un boîtier (101) dans lequel est logé le dispositif de ventilation (10) et pouvant être relié de manière amovible à un récipient de collecte de sécrétion (3).

14. Programme informatique destiné à commander un dispositif de drainage thoracique comportant un récipient de collecte de sécrétion (3), au moins une première soupape (12) pouvant être commandée, au moins un premier capteur de pression (11) et un dispositif de commande (7), dans lequel le programme informatique, lors de son exécution, amène le dispositif de commande (7) à mettre en oeuvre les étapes suivantes :
- lire le premier capteur de pression (11) afin d'obtenir une pression régnant dans le récipient de collecte de sécrétion (3) ; et
**caractérisé par** l'étape consistant à :
- commander la première soupape (12) pour éliminer l'air du récipient de collecte de sécrétion (3) lorsque la différence entre la pression régnant dans le récipient de collecte de sécrétion (3) et la pression atmosphérique dépasse une première valeur de seuil positive, dans lequel la valeur de seuil se situe entre 0,001 et 0,05 kPa.

15. Programme informatique selon la revendication 14, dans lequel le programme informatique, lors de son exécution, amène le dispositif de commande (7) à exécuter en outre les étapes suivantes :
- obtenir la différence entre la pression au niveau du connecteur à vide (25) du dispositif de drainage thoracique et la pression atmosphérique ;
- ouvrir automatiquement une deuxième soupape (22) pouvant être commandée, disposée entre le récipient de sécrétion et le connecteur à vide lorsque ladite différence s'abaisse en dessous d'une valeur limite prédéterminée.
